# EUROPEAN PATENT SPECIFICATION

(11) **EP 3 510 997 B1**
(45) Date of publication and mention of the grant of the patent: **05.08.2020**
(21) Application number: 18386001.4
(22) Date of filing: 10.01.2018
(51) Int. Cl.: A61K 9/48, A61K 31/4985, A61K 9/10

(54) **SOFT GELATIN CAPSULES COMPRISING A SUSPENSION OF TADALAFIL**
WEICHGELATINEKAPSELN MIT EINER SUSPENSION FÜR TADALAFIL
CAPSULES DE GÉLATINE MOLLE CONTENANT UNE SUSPENSION DE TADALAFIL

(43) Date of publication of application: 17.07.2019
(73) Proprietor: Gap S.A., 173 41 Agios Dimitrios Attikis (GR)
(72) Inventor: Panethymitaki, Chrysoula, 145 76 Dionyssos Athens (GR); Georgikopoulou, Kalliopi, 167 77 Elliniko Athens (GR); Pitsikas, Georgios Taxiarhis, 104 44 Athens (GR)
(74) Representative: Roukounas, Dimitrios

(56) References cited:
- WO-A1-2017/196148

## Description

### Technical field of the invention

The present invention relates to soft gelatine capsules comprising tadalafil, or a pharmaceutically acceptable salt thereof.

### Background of the invention

Type 5 Phosphodiesterase (PDE-5) is an enzyme that breaks down cyclic guanoside monophosphate (cGMP), which when inhibited enhances the vasodilatory effect. It is expressed in penile corpus cavernosum and therefore PDE-5 is a target for the treatment of erectile dysfunction. Its inhibitors, such as tadalafil, sildenafil and vardenafil have been studied extensively for the treatment of erectile dysfunction.

Patent application WO9919978 discloses a series of tetracyclic derivatives which are potent and selective inhibitors of cGMP-specific PDE that have found use in a wide range of therapeutic areas, including erectile dysfunction and generally vascular diseases, such as pulmonary hypertension. Patent application WO9703675 discloses the specific use of cGMP phosphodiesterase inhibitors, including tadalafil, for the prophylactic and curative treatment of erectile dyscfunction.

Tadalafil is a carboline derivative and phosphodiesterase 5 inhibitor marketed by Eli Lilly in tablet form under the trade name CIALIS(R) for the treatment of erectile dysfunction and under the trade name ADCIRCA(R) for the treatment of pulmonary arterial hypertension. Its chemical name is (6R,12aR)-6-(Benzo[d][1,3]dioxol-5-yl)-2-methyl-2,3,12,12a and its chemical structure is shown below (CAS# 171596-29-5).

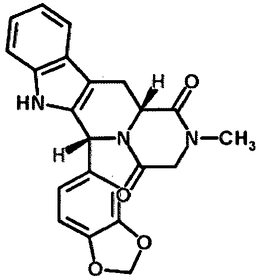

Tadalafil is practically insoluble in water and very slightly soluble in ethanol and other organic solvents. Its low water solubility makes it a compound with low dissolution rate and low and inconsistent bioavailability. The absolute bioavailability of tadalafil has not been determined.

The poor solubility that characterise many beta-carbolines, including tadalafil, together with the fact that a rapid initiation of action is preferred due to the nature of its indication, has initiated numerous studies around those molecules. For example, there are publications investigating the effect of the particle size of tadalafil in the solubility and bioavailability of the active compound, as well as investigation of tablet formulations, solid dispersion preparations and formulations for hard and soft gelatine capsules, in an attempt to increase dissolution rate.

WO0108688 discloses tadalafil tablets comprising particles having D90<40µm in size, in order to improve uniformity in pharmaceutical formulation, stability and provide desired bioavailability properties. In particular, they showed that the lower the particle size, the higher the maximum concentration in the bloodstream (Cmax) in volunteers during bioequivalence studies and the smaller the time needed to achieve this (Tmax), by comparing tadalafil with particle sizes of D90=52µm, D90=20µm and D90=8.4µm. As expected, the lower the particle size, the higher the Cmax. Therefore particle size of tadalafil is directly related to the Cmax.

US 5985326 discloses the development of co-precipitate preparations of PDE5 inhibitors, in an attempt to increase their solubility, and further compressing it into tablets for oral administration. Reproducibility, however, has shown to be difficult for such preparations. Another disadvantage of this preparation was the delayed Cmax , at about 3-4 hours.

Soft gelatine capsules (SGC) provide advantages over other solid oral forms such as tablets. A soft gelatine capsule is a pharmaceutical form preferred by patients as it is easier to swallow compared to tablets. SGC formulations are particularly preferred for active pharmaceutical ingredients that require fast release in the stomach, as SGC may rupture within 1 and 10 minutes in the stomach and further succeed in fast release of the fill material. In particular, SGC formulation is preferred when used with active pharmaceutical ingredients that have very low solubility in water and is also ideal for solution formulations.

WO0108687 discloses soft gelatin capsules comprising a solution formulation of tadalafil with polyethylene glycol 400 (PEG400 NF) and propylene glycol, with active ingredient at 2-2.8% w/w solution (which corresponds to 714-1000mg fill formulation and approximate capsules size of Oval 18) that presents very high dissolution release at 10min (>90%). In addition, WO0108687 discloses a suspension formulation where the compound is present at 6.25% w/w in a soft gelatin capsule (300mg, approximate capsules size Oval 6), but requires >30min to reach 90% dissolution rate (average dissolution, n=6, at 10min=63%).

WO2017196148 discloses compositions comprising tadalafil and dutasteride, a fatty acid ester derivative of glycerol or a fatty acid ester derivative of propylene glycol and optionally a surfactant. The fatty ester derivative dissolves dutasteride and disperses tadalafil. In a specific embodiment the document discloses a composition comprising dutasteride, tadalafil, glyceryl caprylate/caprate, polyoxyl 40 hardened castor oil, lauroyl macrogol glyceride, poloxamer 124 and butylhydroxytoluene.

When used for the treatment of erectile dysfunction, PDE5 inhibitor formulations are desired to be easily swallowed and have a rapid effect, therefore rapid solubility with potentially rapid bioavailability. The soft gelatin capsules of the prior art comprising a suspension of tadalafil do not exhibit such characteristics. On the other hand, the soft gelatin capsules of the prior art comprising a solution of tadalafil exhibit high dissolution rates but, due to the low solubility of tadalafil, the size of the capsule is significantly larger. The administration of a capsule of smaller size is much easier for patients. Thus, there is a need for the development of soft gelatin capsules comprising a suspension of tadalafil, or a pharmaceutically acceptable salt thereof, of smaller size, exhibiting rapid dissolution and excellent physical and chemical stability.

### Summary of the invention

The present invention provides a SGC comprising a suspension composition of tadalafil, or a pharmaceutically acceptable salt thereof, in a liquid carrier, wherein the liquid carrier comprises a vegetable oil, a non-ionic emulsifier with Hydrophile-Lipophile Balance (HLB) value lower or equal to 10, a non-ionic emulsifier with HLB value from 12 to 14, and one or more thickening agents.

The present invention provides a suspension composition in a SGC with dissolution rate as provided by a solution formulation of tadalafil in SGC described in WO0108687, and therefore a potentially faster onset of action, yet prepared in a smaller capsule size. A SGC according to the present invention has also excellent chemical stability of the active ingredient when tested under stressed conditions. Furthermore, in the SGC of the present invention the particle size and the particle shape of the active ingredient remain unchanged under stressed conditions. This in turn has a positive impact on the bioavailability of the active ingredient.

### Brief description of the drawings

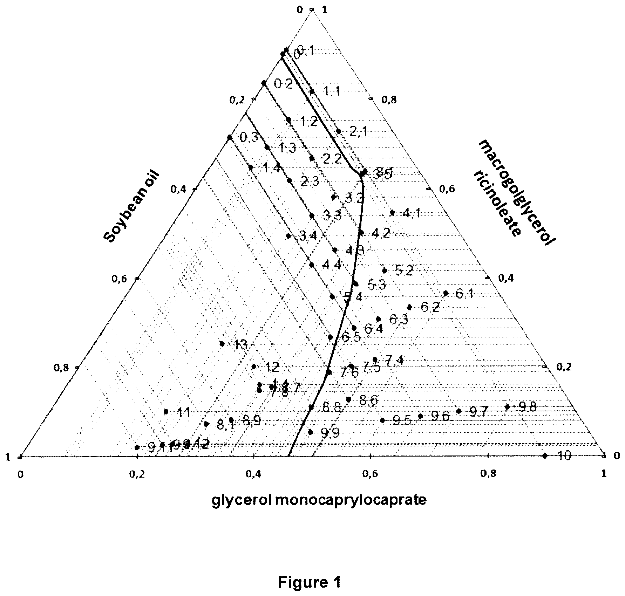

Figure 1 shows a ternary diagram displaying the proportions of three excipients, soybean oil, glycerol monocaprylocaprate and macrogolglycerol ricinoleate and possible combinations of them.

### Detailed description of the invention

For the purposes of the present invention, the following terms have the following meanings:
The term "suspension" refers to a heterogeneous mixture of solid particles and liquid.

The term "emulsifier" refers to a substance that contains a hydrophilic head and a hydrophobic tail and is used to reduce the surface tension between water and oil and subsequently stabilize an oil-in-water or water-in-oil emulsion.

The term "HLB value" refers to Hydrophile-Lipophile Balance and is a measure of hydrophilicity or lipophilicity of an emulsifier.

The term "% w/w" for a given ingredient refers to mg of the ingredient per 100mg of the composition.

The present invention provides a SGC comprising a suspension composition of tadalafil, or a pharmaceutically acceptable salt thereof, in a liquid carrier, wherein the liquid carrier comprises a pharmaceutically acceptable vegetable oil, a non-ionic emulsifier with HLB value lower or equal than 10, a non-ionic emulsifier with HLB value from 12 to 14, and one or more thickening agents.

Preferably, the concentration of tadalafil or a pharmaceutically acceptable salt thereof in the suspension composition is 2-20% w/w. More preferably, the concentration of tadalafil or a pharmaceutically acceptable salt thereof in the suspension composition is 2-10% w/w. Even more preferably, the concentration of tadalafil or a pharmaceutically acceptable salt thereof in the suspension composition is 2-5% w/w.

Preferably, the concentration of the vegetable oil in the suspension composition is 5-45% w/w.

Preferably, the concentration of the non-ionic emulsifier with HLB value lower or equal to 10 in the suspension composition is 5-80% w/w.

Preferably, the concentration of the non-ionic emulsifier with HLB value from 12 to 14 in the suspension composition is 5-80% w/w.

Preferably, the total concentration of the one or more thickening agents in the suspension composition is 0.5-10 %w/w. More preferably, the total concentration of the one or more thickening agents in the suspension composition is 0.5-6%w/w.

Preferably, the SGC of the invention comprises tadalafil.

Examples of a vegetable oil according to the present invention include sunflower oil, safflower oil, soybean oil, maize oil, palm oil, coconut oil. Preferably, the vegetable oil is soybean oil.

Examples of a non-ionic emulsifier with HLB value lower or equal to 10 according to the present invention include, among others, mono and/or di-glycerides of fatty acids (for example glyceryl monocaprylocaprate, glyceryl mono stearate, glyceryl mono-oleate, glyceryl dilaurate), esters of mono- and/or di-glycerides of fatty acids (for example mono/di-succinylated monoglycerides, glyceryl stearate citrate, caprylic/capric diglyceryl succinate), polyglycerol esters of fatty acids (for example polyglyceryl oleate), sorbitan esters of fatty acids (for example sorbitan monooleate, sorbitan monolaurate, sorbitan monostearate). Preferably, a non-ionic emulsifier with HLB value lower or equal to 10 is glyceryl monocaprylocaprate.

Examples of non-ionic emulsifier with HLB value from 12 to 14 according to the present invention include, among others, macrogolglycerol ricinoleate, polysorbates, oleth-10, cocomide and polyethylene glycol (PEG)-8 laurate. Preferably, a non-ionic emulsifier with HLB value from 12 to 14 is macrogolglycerol ricinoleate.

The ratio of the vegetable oil, the non-ionic emulsifier with HLB value lower or equal to 10 and the non-ionic emulsifier with HLB value from 12 to 14 in the compositions of the present invention may vary, provided that the mixture of the three components is uniform and stable.

The suspension composition of the soft gelatin capsule of the present invention comprises one or more thickening agents, which stabilize the suspension. Examples of a thickening agent according to the present invention include, among others, ethoxylated polypropylene glycol, polyalkaline oxide copolymers, lauryl polyoxy glycerides and fumed silica. Preferably, a thickening agent is ethoxylated polypropylene glycol.

In the use of PDE5 inhibitors for the treatment of erectile dysfunction, a fast onset of the action is desirable. Therefore the use of solvents, such as polyethylene glycols, that cause an increase in the rupture time of the SGC is not preferable. The increase in rupture time is often caused by the cross linking of the SGC during time and during stress conditions. Therefore initially it was attempted to create a suspension using vegetable oil as carrier, where the cross-linking phenomenon is minimized.

Solubility studies have shown that tadalafil, is practically insoluble in vegetable oils, such as olive oil, sunflower oil, safflower oil, soybean oil. Suspension formulations of tadalafil were initially produced in soybean oil, and in a mixture of hydrogenated and partially-hydrogenated soybean oil in order to produce a stable suspension. One such example is shown in Table 1. The final suspension produced was stable for 24h and exhibited the dissolution rate shown in Table 2:

**Table 1**

| **Ingredients** | **Amount (mg/cps)** |
|---|---|
| Tadalafil | 20.0 |
| Soybean oil | 352.0 |
| Partially hydrogenated soybean oil | 98.8 |
| fully hydrogenated soybean oil | 24.6 |
| Beeswax | 24.6 |
| Total Fill | 520 |

**Table 2**

| **Time (min)** | **Dissolution rate (ave n=6)** |
|---|---|
| 10 | 2.4 % |
| 20 | 2.8% |
| 30 | 3.1% |
| 60 | 2.9 % |

It is clear that a suspension using soybean oil fails the dissolution profile specifications for an immediate release product.

On the other hand, it has unexpectedly been found that a suspension of tadalafil or a pharmaceutically acceptable salt thereof in a liquid carrier comprising a vegetable oil, a non-ionic emulsifier with HLB value lower or equal to 10 and a non-ionic emulsifier with HLB value from 12 to 14 provide a stable suspension, exhibiting surprisingly high dissolution rate of the active ingredient combined with excellent chemical and particle size stability of the active ingredient.

The suspension composition of the SGC of the present invention can be prepared by processes well known in the art. For example, it can be prepared by mixing all oily ingredients at a temperature above their melting point. The mixing procedure continues under vacuum until a clear and homogeneous mixture is achieved. Then, a step-wise cooling system is in place to drop the temperature to the melting point of the oil mixture. This temperature point is directly dependent on the amount and type of stabilizing agents present in the formulation. The active ingredient is then added, followed by mixing and a step-wise cooling system comes in place again, until the temperature reaches about 17-25°C, or when the suspension has the desired viscosity for encapsulation.

SGC manufacture is an already well-known art and may be performed, for example, by following the process described in US 4744988 A.

The suspension compositions of the SGC of the present invention exhibit surprisingly high dissolution rate of the active ingredient. Such dissolution rate is similar to the dissolution rate of a solution and it is totally unexpected for a suspension composition. Due to the low solubility of tadalafil, its concentration in a suspension composition can be much higher compared to a solution composition. This means that a SGC according to the invention can comprise lower volumes of the liquid carrier compared to a SGC comprising a solution of tadalafil. This in turn means that the size of the capsule can be much smaller, which results in ease of use and better compliance by the patients.

The suspension compositions of the SGC of the present invention exhibit also excellent chemical stability of the active ingredient.

It is already known by a person skilled in the art that smaller particle size of an active ingredient enhances its bioavailability, by enhancing its solubility. In the case of tadalafil this is disclosed in WO0108688.

In the suspension compositions of the SGC of the present invention the shape and size of the active ingredient remain unchanged under stressed conditions. This means that the bioavailability of the active ingredient remains unchanged throughout the shelf life of the pharmaceutical product.

Therefore, the present invention provides a SGC which exhibits fast solubilisation of the active ingredient when in dissolution medium and potentially increases bioavailability while maintaining its crystal size and shape.

### Examples

### Example 1

### Testing of suspension excipient formulations

Suspension excipient formulations were investigated for their dissolution, using soybean oil as a vegetable oil,
glycerol monocaprylocaprate as a non-ionic emulsifier with HLB value lower or equal to 10
macrogolglycerol ricinoleate as a non-ionic emulsifier with HLB value from 12 to 14.

The samples were maintained at room temperature for 7 days and were evaluated for their stability. The results are demonstrated with the ternary diagram in Figure 1. A ternary diagram displays the proportion of 3 variables, soybean oil, glycerol monocaprylocaprate and macrogolglycerol ricinoleate and one may visualize all possible combinations of these ingredients. The area on the right hand side of the diagram's curve represents the different combinations of the three ingredients that provide a stable oil mixture.

Samples of stable oil mixtures, as described in the ternary diagram, were further formulated with tadalafil as active ingredient at a ratio of 1:25 (tadalafil: excipients), which was then stabilized by using thickening agents. The tadalafil active ingredient used in the experiments has particle size D90<10µm. The release of tadalafil was evaluated using in vitro dissolution test, using USP Type-II apparatus (paddle) at 75rpm in 1000ml of 0.5% w/v sodium dodecyl sulfate solution in water at 37°C ±5°C. The formulations tested as well as the results from the in vitro dissolution are shown in Table 3.

**Table 3**

| **Ingredients** | **Samples** | | | | | | |
|---|---|---|---|---|---|---|---|
| | **1.1** | **4.2** | **5.3** | **6.5** | **7.6** | **8.8** | **9.9** |
| | mg/cps | | | | | | |
| Tadalafil | 20 | 20 | 20 | 20 | 20 | 20 | 20 |
| Soybean oil | 44 | 81 | 113 | 162 | 239 | 289 | 351.5 |
| Glycerol monocaprylocaprate | 44 | 162 | 186 | 194 | 172 | 157 | 120 |
| Macrogolglycerol ricinolate | 396 | 242 | 186 | 129 | 74 | 39 | 13.5 |
| Thickener | 15 | 15 | 15 | 15 | 15 | 15 | 15 |
| | | | | | | | |
| Total fill weight | 520 | 520 | 520 | 520 | 520 | 520 | 520 |
| | | | | | | | |

| **Dissolution** Water/ 0.5% SDS | | | | | | | |
|---|---|---|---|---|---|---|---|
| 10min | 98.6% | 92.5% | 95.6% | 99.9% | 96.8% | 99.6% | 85.5% |

### Example 2

The suspension composition shown in Table 4 (composition 5.3 of Table 3) was filled in soft gelatin capsules and investigated for its chemical stability, release and particle size of the active ingredient.

**Table 4**

| **Ingredients** | **Amount (mg/cps)** |
|---|---|
| Tadalafil | 20.0 |
| Soybean oil | 113.0 |
| glycerol monocaprylocaprate | 186 |
| macrogolglycerol ricinoleate | 186 |
| ethylene oxide/propylene oxide | 14.6 |
| lauryl macrogol glycerides | 0.4 |
| Total Fill | 520.0 |

Encapsulation of the suspension was performed using oval shape, size 10 capsule, using standard techniques known to a person skilled in the art.

The soft gelatine capsules produced were stored at 25°C, 30°C for 12 months and 40°C for 6 months and their stability in terms of particle size of the active compound, assay content, related substance and dissolution has been examined, as compared to fresh capsules (Bulk, 0 time). Particle size distribution (PSD) was also performed using MALVERN MASTERSIZER/S analyser.

The nomenclature for particle size is described and used herein as D90, D50, D10, where D90 of 8.06 µm means that at least 90% of the particles in the samples have a particle size less than 8.06µm. D[4,3] is the volume mean diameter of the particles. The measurements of PSD are summarised in Table 5.

**Table 5**

| | **Particle size Distribution** | | | |
|---|---|---|---|---|
| | **Bulk (0 time)** | **25°C/ 60%RH** | **30°C/ 65%RH** | **40°C/ 75%RH** |
| **D10/µm** | 1.04 | 1.08 | 1.02 | 1.04 |
| **D50/µm** | 3.76 | 4.03 | 4.06 | 3.73 |
| **D90/µm** | 8.06 | 8.38 | 8.64 | 8.33 |
| **D[4.3]/µm** | 4.25 | 4.47 | 4.55 | 4.31 |

The results of Table 5 demonstrate that the particle size of tadalafil in the suspension remains unchanged. As discussed before, the particle size of tadalafil is directly related to its bioavailability, therefore the composition will not alter solubility and bioavailability data under stressed conditions.

The capsules stored under stressed conditions for 12 months were further evaluated for their dissolution properties. The release of tadalafil was evaluated using in vitro dissolution test, using USP Type-II apparatus (paddle) at 75rpm in 1000ml of 0.5% w/v sodium dodecyl sulfate solution in water at 37°C ±5°C.The results are summarised in Table 6 below and show that rupture of the SGC and subsequent solubility of the active ingredient remains unchanged.

**Table 6**

| | **Dissolution Profile (average, n=6)** | | | |
|---|---|---|---|---|
| | **Bulk (0 time)** | **25°C/ 60%RH** | **30°C/ 65%RH** | **40°C/ 75%RH** |
| **10min** | 95.5 | 95.3 | 95.5 | 94.4 |
| **20min** | 98.3 | 97.3 | 98.1 | 95.0 |
| **30min** | 98.5 | 97.6 | 98.9 | 97.7 |

In addition, the chemical stability results of the capsules manufactured as described above are shown in Table 7 below.

**Table 7**

| | **Product Chemical Stability** | |
|---|---|---|
| | **Assay (%)** | **Related Substances (total, %)** |
| **Bulk** | 100.1% | BQL |
| **25°C/ 60%RH** | 98.9% | BQL |
| **30°C/ 65%RH** | 99.4% | BQL |
| **40°C/ 75%RH** | 99.5% | BQL |

| | | |
|---|---|---|
| BQL: Below Quantifiable Limit | | |

The results show that the excipients used maintain the active ingredient's crystals size and shape within the composition stable and unchanged, when stored in stability chambers of 25°C, 30°C for 12 months and 40°C for 6 months, as compared to the fresh capsules.

## Claims

1. A soft gelatin capsule comprising a suspension composition of tadalafil, or a pharmaceutically acceptable salt thereof, in a liquid carrier, wherein the liquid carrier comprises a vegetable oil, a non-ionic emulsifier with hydrophile-lipophile balance value lower or equal to 10, a non-ionic emulsifier with hydrophile-lipophile balance value from 12 to 14, and one or more thickening agents.

2. A soft gelatin capsule according to claim 1 comprising tadalafil.

3. A soft gelatin capsule according to claim 1 or 2, wherein the vegetable oil is selected from the group consisting of sunflower oil, safflower oil, soybean oil, maize oil, palm oil and coconut oil.

4. A soft gelatin capsule according to claim 3, wherein the vegetable oil is soybean oil.

5. A soft gelatin capsule according any one of the preceding claims, wherein the non-ionic emulsifier with hydrophile-lipophile balance value lower or equal to 10 is selected from the group consisting of mono and/or di-glycerides of fatty acids, esters of mono- and/or di-glycerides of fatty acids, polyglycerol esters of fatty acids and sorbitan esters of fatty acids.

6. A soft gelatin capsule according to any one of the preceding claims, wherein the non-ionic emulsifier with hydrophile-lipophile balance value lower or equal to 10 is selected from the group consisting of glyceryl monocaprylocaprate, glyceryl mono stearate, glyceryl mono-oleate, glyceryl dilaurate, mono/di-succinylated monoglycerides, glyceryl stearate citrate, caprylic/capric diglyceryl succinate, polyglyceryl oleate, sorbitan monooleate, sorbitan monolaurate and sorbitan monostearate.

7. A soft gelatin capsule according claim 6, wherein the non-ionic emulsifier with hydrophile-lipophile balance value lower or equal to 10 is glyceryl monocaprylocaprate.

8. A soft gelatin capsule according any one of the preceding claims, wherein the non-ionic emulsifier with hydrophile-lipophile balance value from 12 to 14 is selected from the group consisting of macrogolglycerol ricinoleate, polysorbates, oleth-10, cocomide and PEG-8 laurate.

9. A soft gelatin capsule according claim 8, wherein the non-ionic emulsifier with hydrophile-lipophile balance value from 12 to 14 is macrogolglycerol ricinoleate.

10. A soft gelatin capsule according to any one of the preceding claims, wherein the one or more thickening agents are selected from the group consisting of ethoxylated polypropylene glycol, polyalkaline oxide copolymers, lauryl macrogol glycerides, fumed silica and mixtures thereof.

11. A soft gelatin capsule according to claim 9, wherein the one or more thickening agents is selected from the group consisting of ethoxylated polypropylene glycol, lauryl macrogolglycerides and mixtures thereof.

12. A soft gelatin capsule according to any one of the preceding claims, wherein the concentration of tadalafil or a pharmaceutically acceptable salt thereof is 2-20% w/w.

13. A soft gelatin capsule according to any one of the preceding claims, wherein the concentration of the vegetable oil in the suspension composition is 5-45% w/w.

14. A soft gelatin capsule according to any one of the preceding claims, wherein the concentration of the non-ionic emulsifier with hydrophile-lipophile balance value lower or equal to 10 in the suspension composition is 5-80% w/w.

15. A soft gelatin capsule according to any one of the preceding claims, wherein the concentration of the non-ionic emulsifier with hydrophile-lipophile balance value from 12 to 14 in the suspension composition is 5-80% w/w.

16. A soft gelatin capsule according to any one of the preceding claims, wherein the concentration of the one or more thickening agents in the suspension composition is 0.5-10 %w/w.

## Patentansprüche

1. Weichgelatinekapseln, umfassend eine Suspensionszusammensetzung von Tadalafil, oder einem pharmazeutisch verträglichen Salz davon, in einem flüssigen Träger, wobei der flüssige Träger ein Pflanzenöl, einen nicht-ionischen Emulgator mit hydrophil-lipophilem Gleichgewichtswert von kleiner oder gleich 10, einen nicht-ionischen Emulgator mit hydrophil-lipophilem Gleichgewichtswert von 12 bis 14 und ein oder mehrere Verdickungsmittel umfasst.

2. Weichgelatinekapsel gemäß Anspruch 1, umfassend Tadalafil.

3. Weichgelatinekapsel gemäß Anspruch 1 oder 2, wobei das Pflanzenöl ausgewählt ist aus der Gruppe bestehend aus Sonnenblumenöl, Distelöl, Sojabohnenöl, Maisöl, Palmöl und Kokosnussöl.

4. Weichgelatinekapsel gemäß Anspruch 3, wobei das Pflanzenöl Sojabohnenöl ist.

5. Weichgelatinekapsel gemäß einem der vorangehenden Ansprüche, wobei der nicht-ionische Emulgator mit hydrophil-lipophilem Gleichgewichtswert von kleiner oder gleich 10 ausgewählt ist aus der Gruppe bestehend aus Mono- und/oder Diglyceriden von Fettsäuren, Estern von Mono- und/oder Diglyceriden von Fettsäuren, Polyglycerolestern von Fettsäuren und Sorbitanestern von Fettsäuren.

6. Weichgelatinekapsel gemäß einem der vorangehenden Ansprüche, wobei der nicht-ionische Emulgator mit hydrophil-lipophilem Gleichgewichtswert von kleiner oder gleich 10 ausgewählt ist aus der Gruppe bestehend aus Glycerylmonocaprylocaprat, Glycerylmonostearat, Glycerylmonoleat, Glyceryldilaurat, mono/di-succinylierten Monoglyceriden, Glycerylstearatcitrat, Capryl-/ Caprindiglycerylsuccinat, Polyglyceryloleat, Sorbitanmonooleat, Sorbitanmonolaurat und Sorbitanmonostearat.

7. Weichgelatinekapsel gemäß Anspruch 6, wobei der nicht-ionische Emulgator mit hydrophil-lipophilem Gleichgewichtswert von kleiner oder gleich 10 Glycerylmonocaprylocaprat ist.

8. Weichgelatinekapsel gemäß einem der vorangehenden Ansprüche, wobei der nicht-ionische Emulgator mit hydrophil-lipophilem Gleichgewichtswert von 12 bis 14 ausgewählt ist aus der Gruppe bestehend aus Macrogolglycerolricinoleat, Polysorbaten, Oleth-10, Cocamid und PEG-8-Laurat.

9. Weichgelatinekapsel gemäß Anspruch 8, wobei der nicht-ionische Emulgator mit hydrophil-lipophilem Gleichgewichtswert von 12 bis 14 Macrogolglycerolricinoleat ist.

10. Weichgelatinekapsel gemäß einem der vorangehenden Ansprüche, wobei das eine oder die mehreren Verdickungsmittel ausgewählt sind aus der Gruppe bestehend aus ethoxyliertem Polypropylenglykol, Polyalkylenoxid-Copolymeren, Laurylmacrogolglyceriden, pyrogener Kieselsäure und Gemischen davon.

11. Weichgelatinekapsel gemäß Anspruch 9, wobei das eine oder die mehreren Verdickungsmittel ausgewählt sind aus der Gruppe bestehend aus ethoxyliertem Polypropylenglykol, Laurylmacrogolglyceriden und Gemischen davon.

12. Weichgelatinekapsel gemäß einem der vorangehenden Ansprüche, wobei die Konzentration von Tadalafil oder einem pharmazeutisch verträglichen Salz davon 2-20% w/w beträgt.

13. Weichgelatinekapsel gemäß einem der vorangehenden Ansprüche, wobei die Konzentration des Pflanzenöls in der Suspensionszusammensetzung 5-45% w/w beträgt.

14. Weichgelatinekapsel gemäß einem der vorangehenden Ansprüche, wobei die Konzentration des nicht-ionischen Emulgators mit hydrophil-lipophilem Gleichgewichtswert von kleiner oder gleich 10 in der Suspensionszusammensetzung 5-80% w/w beträgt.

15. Weichgelatinekapsel gemäß einem der vorangehenden Ansprüche, wobei die Konzentration des nicht-ionischen Emulgators mit hydrophil-lipophilem Gleichgewichtswert von 12 bis 14 in der Suspensionszusammensetzung 5-80% w/w beträgt.

16. Weichgelatinekapsel gemäß einem der vorangehenden Ansprüche, wobei die Konzentration des einen oder der mehreren Verdickungsmittels in der Suspensionszusammensetzung 0,5-10% w/w beträgt.

## Revendications

1. Capsule en gélatine molle comprenant une composition de tadalafil en suspension, ou un sel pharmaceutiquement acceptable de celui-ci, dans un véhicule liquide, dans laquelle le véhicule liquide comprend une huile végétale, un émulsifiant non ionique avec une valeur d'équilibre hydrophile-lipophile inférieure ou égale à 10, un émulsifiant non ionique avec une valeur d'équilibre hydrophile-lipophile de 12 à 14, et un ou plusieurs agents épaississants.

2. Capsule en gélatine molle selon la revendication 1 comprenant du tadalafil.

3. Capsule en gélatine molle selon la revendication 1 ou 2, dans laquelle l'huile végétale est sélectionnée dans le groupe consistant en l'huile de tournesol, l'huile de carthame, l'huile de soja, l'huile de maïs, l'huile de palme et l'huile de coco.

4. Capsule en gélatine molle selon la revendication 3, dans laquelle l'huile végétale est l'huile de soja.

5. Capsule de gélatine molle selon l'une quelconque des revendications précédentes, dans laquelle l'émulsifiant non ionique avec une valeur d'équilibre hydrophile-lipophile inférieure ou égale à 10 est sélectionné dans le groupe consistant en les mono- et/ou di-glycérides d'acides gras, les esters de mono- et/ou di-glycérides d'acides gras, les esters de polyglycérol d'acides gras et les esters de sorbitane d'acides gras.

6. Capsule de gélatine molle selon l'une quelconque des revendications précédentes, dans laquelle l'émulsifiant non ionique avec une valeur d'équilibre hydrophile-lipophile inférieure ou égale à 10 est sélectionné dans le groupe consistant en le monocaprylocaprate de glycéryle, le monostéarate de glycéryle, le monooléate de glycéryle, le dilaurate de glycéryle, les monoglycérides mono/di-succinylés, le citrate de stéarate de glycéryle, le succinate de diglycéryle caprylique/caprique, l'oléate de polyglycéryle, le monooléate de sorbitane, le monolaurate de sorbitane et le monostéarate de sorbitane.

7. Capsule en gélatine molle selon la revendication 6, dans laquelle l'émulsifiant non ionique avec une valeur d'équilibre hydrophile-lipophile inférieure ou égale à 10 est le monocaprylocaprate de glycéryle.

8. Capsule de gélatine molle selon l'une quelconque des revendications précédentes, dans laquelle l'émulsifiant non ionique avec une valeur d'équilibre hydrophile-lipophile de 12 à 14 est sélectionné dans le groupe consistant en le ricinoléate de macrogolglycérol, les polysorbates, l'oleth-10, le cocomide et le laurate de PEG-8.

9. Capsule en gélatine molle selon la revendication 8, dans laquelle l'émulsifiant non ionique avec une valeur d'équilibre hydrophile-lipophile de 12 à 14 est le ricinoléate de macrogolglycérol.

10. Capsule de gélatine molle selon l'une quelconque des revendications précédentes, dans laquelle les un ou plusieurs agents épaississants sont sélectionnés dans le groupe consistant en le polypropylène glycol éthoxylé, les copolymères d'oxyde polyalcalin, les glycérides de lauryl macrogol, la silice fumée et les mélanges de ceux-ci.

11. Capsule en gélatine molle selon la revendication 9, dans laquelle les un ou plusieurs agents épaississants sont sélectionnés dans le groupe consistant en le polypropylène glycol éthoxylé, les glycérides de lauryl macrogol et les mélanges de ceux-ci.

12. Capsule de gélatine molle selon l'une quelconque des revendications précédentes, dans laquelle la concentration en tadalafil ou un sel pharmaceutiquement acceptable de celui-ci est de 2 à 20 % p/p.

13. Capsule de gélatine molle selon l'une quelconque des revendications précédentes, dans laquelle la concentration en huile végétale dans la composition en suspension est de 5 à 45 % p/p.

14. Capsule de gélatine molle selon l'une quelconque des revendications précédentes, dans laquelle la concentration en émulsifiant non ionique avec une valeur d'équilibre hydrophile-lipophile inférieure ou égale à 10 dans la composition en suspension est de 5 à 80 % p/p.

15. Capsule de gélatine molle selon l'une quelconque des revendications précédentes, dans laquelle la concentration en émulsifiant non ionique avec une valeur d'équilibre hydrophile-lipophile de 12 à 14 dans la composition en suspension est de 5 à 80 % p/p.

16. Capsule de gélatine molle selon l'une quelconque des revendications précédentes, dans laquelle la concentration des un ou plusieurs agents épaississants dans la composition en suspension est de 0,5 à 10 % p/p.
